# EUROPEAN PATENT APPLICATION

(11) **EP 3 167 910 A1**
(43) Date of publication of application: **17.05.2017**
(21) Application number: 15193976.6
(22) Date of filing: 10.11.2015
(51) Int. Cl.: A61L 27/24, A61L 27/56, A61L 27/60

(54) **METHOD FOR PRODUCING A BIOCOMPATIBLE MATRIX WITH TARGETED BIOMECHANICAL PROPERTIES**

(71) Applicant: MedSkin Solutions Dr. Suwelack AG, 48727 Billerbeck (DE)
(72) Inventor: Behrens, Daniel Timo, 48301 Nottuln (DE); Doberenz, Claudia, 48149 Münster (DE); Cassing, Anke, 45481 Mülheim (DE); Kassner, Anja, 48161 Münster (DE); Schmeing, Stefanie, 48653 Coesfeld (DE); Kunz, Michael, 48153 Münster (DE)
(74) Representative: CH Kilger Anwaltspartnerschaft mbB

(57) **Abstract**

Biopolymer-based matrices are an important basis for many cosmetic products or medical devices, such as implants, wound dressings or facial masks. It is therefore essential that the properties of the matrices are adaptable to the intended use. The present invention relates to a method for the production of a biocompatible matrix with adaptable properties the method comprising:
a) providing a composition comprising at least one biopolymer;
b) extruding the composition into a layer through a slit onto a surface, wherein the slit moves over the surface;
c) freezing the composition after extrusion;
d) optionally repeating the process to add one or more further layers;
e) lyophilizing the frozen composition;
f) optionally subjecting the composition to mechanical force.
g) crosslinking the at least one biopolymer;

wherein preferably the surface can be cooled.

The invention further relates to a biocompatible matrix obtainable by the method as well as to the use of a matrix obtainable by the method for medical or cosmetic purposes.

## Description

### FIELD OF THE INVENTION

The present invention is in the field of biopolymers, in particular in the field of biopolymer-based matrices. Furthermore, the invention relates to the production of biopolymer-based matrices and the use of biopolymer-based matrices, in particular the medical and cosmetic use of biopolymer-based matrices.

### BACKGROUND

Biopolymer based matrices are an essential basis for several medical and cosmetic products. In the medical area, said matrices form the basis for products comprising wound dressings, biocompatible implants, implantable scaffolds for tissue regeneration and/or other medical devices. For cosmetical use, said matrices form the basis for products comprising cosmetic masks, gels and/or thickening agents in serums or creams.

Many of these biopolymer-based matrices are based on the biopolymer collagen. Alternative biopolymers include hyaluronic acid, elastin, fibronectin, perlecan, aggrecan or laminin. The properties and uses of said matrices vary, depending on the biopolymer used as basis.

Biopolymer-based matrices have several advantages compared to matrices based on artificial materials such as silicone, polyesters or poly(meth)acrylic polymers, which provide a structural and/or mechanical function without inducing foreign body response but do not provide any biological stimulation of the organism to interact with the scaffold (e.g. cell colonization). Matrices, based on artificial polymers, such as silicones, allow a better control over the polymer properties, based on the composition of the artificial polymer. Several properties of the matrix can be controlled.

The use of biopolymers in a matrix is challenging due to the complexity of the biological system. Therefore, one task of the present invention was the provision of a method for the generation of biopolymer-based matrices with improved properties by using the natural inherent structural advantages.

### BRIEF DESCRIPTION OF THE INVENTION

The present invention relates to a method for the production of a biocompatible matrix with adaptable properties the method comprising:
a) providing a composition comprising at least one biopolymer;
b) extruding the composition into a layer through a slit onto a surface, wherein the slit moves over the surface;
c) freezing the composition after extrusion;
d) optionally repeating the process to add one or more further layers;
e) lyophilizing the frozen composition;
f) optionally subjecting the composition to mechanical force.
g) crosslinking the at least one biopolymer;
wherein preferably the surface can be cooled.

The invention further relates to a biocompatible matrix obtainable by the method as well as to the use of a matrix obtainable by the method for medical or cosmetic purposes.

### DETAILED DESCRIPTION OF THE INVENTION

Biopolymer-based matrices are an important basis for many cosmetic or medical products, such as implants, wound dressings or facial masks. It is therefore essential that the properties of the matrices are adaptable to the intended use. The inventors have developed a method, which allows adapting the properties of a biopolymer-based matrix easily for many different applications.

Accordingly, the invention relates to a method for the production of a biocompatible matrix with adaptable and/or improved properties, the method comprising:
a) providing a composition comprising at least one biopolymer;
b) extruding the composition into a layer through a slit onto a surface, wherein the slit moves over the surface;
c) freezing the composition after extrusion;
d) optionally repeating the process to add one or more further layers;
e) lyophilizing the frozen composition;
f) optionally subjecting the composition to mechanical force.
g) crosslinking the at least one biopolymer;
wherein preferably the surface can be cooled.

The inventors surprisingly found that the properties of a biopolymer-based matrix produced with the method can be adjusted by the appropriate selection of the process parameters. With the above method it is possible to directionally adapt tensile strength, biodegradability of the matrix, plastic deformation or the elastic modulus of the biopolymer-based matrix.

In the context of the present invention, biopolymers are polymers produced by living organisms and/or mimicking such. In the present invention the term "biopolymer" encompasses all naturally occurring modifications of biopolymers, e.g. glycosylation, partial hydrolysis or the attachment of lipids to polypeptides, but is not limited to those.

Non-limiting examples for biopolymers according to the present invention comprise: collagens, starch, cellulose derivatives, glycosaminoglycan, alginates, polysaccarides or fucoidanes.

The matrix might be based on any biopolymer. It is however preferred that said at least one biopolymer used in the method comprises a characteristic suprastructural organisation of components, e.g. (micro) fibrils, fibers or networks. In a preferred embodiment of the invention the characteristic suprastructural organisation is a fiber/fibril structure.

The biopolymer is provided in a composition. Said composition might comprise a purified and/or modified biopolymer. However said composition might comprise a native biopolymer. The composition might comprise further components. In one embodiment the composition comprises proteins, glycoproteins, proteoglycans and glucosaminoglycans such as hyaluronic acid. In a preferred embodiment, the composition is a naturally existing composition or derived from a naturally existing composition.

In one embodiment of the invention said composition is or comprises a biologically derived tissue and/or tissue component, comprising said biopolymer. In a preferred embodiment the composition comprising at least one biopolymer is or comprises a biologically derived tissue or tissue component selected from the group comprising bovine, equine, porcine, rodent, piscine tissue, preferably skin, tendon or cartilage tissue, most preferably bovine split skin tissue.

In one embodiment of the invention the biologically derived tissue or tissue component is not modified or processed prior to use. In a preferred embodiment the biologically derived tissue has been processed prior to its use in the method. In one embodiment minor processing, such as addition of water, washing, dilution, and/or chemical treatment on the biologically derived tissue or tissue component has been performed. In an alternative embodiment substantial processing, such as mechanical breakup of the raw material, isolation, purification, addition of water, washing, dilution, chemical treatment with HCl, NaOH, oxidants , pH-adjustment, mechanical treatment and/or mechanical breakup (e.g. by mixing, mincing, milling, cutting, die-cutting), freezing and/or defrosting, has been performed.

In one embodiment of the invention, step b) involves the use of biological derived tissue(s) as composition and extrusion is performed by means of depositing said biological derived tissue(s).

In a preferred embodiment, said deposition is performed involving the folding of said biological derived tissue(s). In a more preferred embodiment, the deposition is performed by means of assembling of said biological derived tissue(s) to a multi-layered three-dimensional structure.

In a most preferred embodiment, said deposition involves mechanical treatment of biological derived tissue(s) by means of planarisation, cutting, die-cutting, embossing, slitting, generation of holes.

In an alternative preferred embodiment, said deposition involves mechanical treatment of biological derived tissue(s) by means of one- or two-dimensional shaping, e.g. in the plane of the matrix or over a 3D-template.

In an alternative embodiment a purified biopolymer is used and the composition is an artificial composition. In one embodiment the composition mimics a biological composition.

In a preferred embodiment of the invention the biopolymer is a biopolymer naturally occurring in a biologically derived tissue. In a more preferred embodiment the biopolymer is selected from the group comprising collagen, elastin, fibronectin, perlecan, aggrecan, laminin, hyaluronic acid.

In the most preferred embodiment of the invention the at least one biopolymer is collagen, more preferably bovine collagen.

In one embodiment of the invention the biopolymer is an artificially or biotechnologically generated analog of the biopolymer.

The biopolymer might be processed with methods known to the person skilled in the art, in order to extract, purify or optimize the biopolymer. Potential treatments include but are not limited to:
- Addition of water
- Chemical treatment such as acid treatment, alkaline or oxidative treatment, with or without mechanical forces (e.g. rotation)
- Washing
- pH-Adjustment
- Mechanical treatment and/or mechanical breakup (e.g. by mixing, mincing, milling, (Die-) cutting)
- Freezing
- Defrosting
- (Die-)Cutting into desired segment or shape

The biopolymer might be processed with a combination of two or more treatments.

In a preferred embodiment of the invention the biopolymer is collagen. In a more preferred embodiment the biopolymer is collagen extracted from bovine dermis. Most preferably the biopolymer has been prepared as follows:
- Isolation and purification of collagen from bovine dermis by chemical treatment with acid, alkaline and/or oxidative agents
- Mincing of chemically treated material
- Milling resulting in a collagen dispersion

In another preferred embodiment, the composition comprises biologically derived tissue that has been prepared as follows:
- Purification of tissue by mild chemical treatment with acid, alkaline and/or oxidative agents
- Optional (Die-)cutting into desired segment or shape
- Optional shaping by mechanical forces (planar or 3D template) and subsequent freezing under force
- Optional mechanical shaping in frozen state, e.g. (cryo) cutting, CNC milling, grinding.

The composition should be preferably a composition with high viscosity. It is not important, whether the composition comprises a naturally high viscosity or if the composition comprises additional compounds which increase the viscosity, such as hyaluronic acid, cellulosic polymers, and synthetic hydrophilic polymers.

In another embodiment, the viscosity is adapted by dilution. In another embodiment the particle size and therefore the viscosity was changed without adding additional components for example by milling.

The composition comprising the at least one biopolymer might comprise further components, which influence the properties of the matrix or contribute to the intended use. Non-limiting examples for suitable additional components are: pharmaceutically and/or cosmetically acceptable dyes and coloring agents, medically active ingredients, compounds that improve the product performance, e.g. biocompatibility, biodegradability, mechanical, sensorial or antioxidative properties, the promotion of wound healing and tissue regeneration, and water binding capacity.

Medically active compounds, suitable for addition to the composition, include, but are not limited to, anesthetics, such as lidocaine and other agents that prevent transmission of nerve impulses, salicylates, diclofenac, analgesics, antibiotics or antibiotic compounds, antimicrobials or antimicrobial compounds such as silver and silver ions, vitamins, antioxidants, and compounds improving self-healing, such as panthenol, biologically active compounds such as growth factors.

The inventors found that the method of deposition of the composition directly influences the physico-chemical properties of the matrix. The inventors surprisingly found that extrusion through a slit, preferably 0.5 to 5 mm wide allows an ordered deposition of the characteristic structure of the biopolymer, which influences the properties of the matrix.

Preferably the slit has a width of 0.5 to 5 mm. In a preferred embodiment the slit has a width between 1 and 3 mm. The length of the slit is variable, preferably in the range 1 to 100 cm.

In one embodiment of the invention the surface is a mold. In a preferred embodiment the mold has a specified topography. In a more preferred embodiment said specified topography comprises one or more than one of the following topological structures: flat surface, planar structure, nonplanar structure, continuous structure, non-continuous structure, fillet, channel, platform, hole, pin, honeycomb, and network.

In a preferred embodiment of the invention the mold additionally allows horizontal rotation to adapt extrusion direction.

In order to deposit the composition on a surface or into a mold, the slit should be moving. The slit should preferably move in a straight line. The thickness of the layer derived from the composition and thereby some properties of the matrix can be controlled with the speed of movement of the slit and the pressure of extrusion.

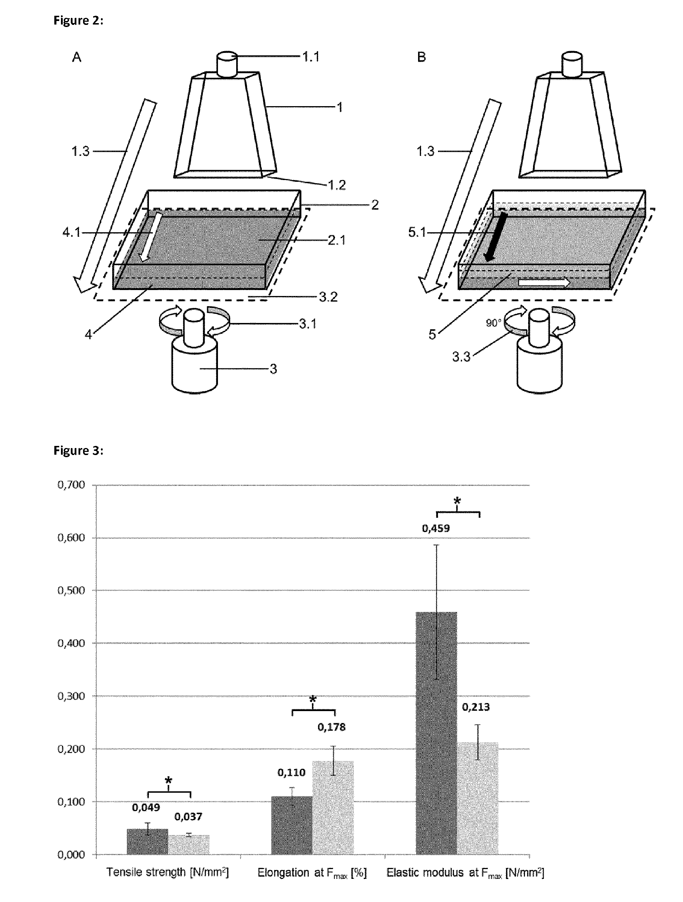

In a preferred embodiment the extrusion is performed in longitudinal orientation to the mold defining the later shape and orientation of the produced biocompatible matrix. In an alternative preferred embodiment the extrusion is performed in orthogonal orientation to the mold defining the later shape and orientation of the produced biocompatible matrix (see figure 2), thus creating a flat, preferably rectangular layer or composition of layers.

In general a faster moving slit will create a thinner layer, while a slow moving slit will produce a thicker layer, provided a constant pressure and flowrate.

It is possible to vary the movement speed of the slit, the size and shape of the slit and the flow rate of the composition, in order to create a matrix with varying thickness, e.g. a matrix comprising predetermined breaking points.

The composition is extruded by application of pressure enabling the extrusion of composition with adaptable flow rates. In one embodiment of the invention the composition is extruded using only gravity pressure, i.e. no application of external pressure. In a preferred embodiment the composition is extruded using mechanical pressure, e.g. by providing a pump. In a more preferred embodiment said pump additionally supplies a steady supply of the composition.

Any method to apply pressure is suitable to extrude the composition, preferably the pressure is controllable.

In one embodiment of the invention the composition is extruded using constant pressure. In an alternative embodiment of the invention the composition is extruded using a variable pressure.

In a most preferred embodiment the thickness of the layer of the composition is controlled by a combination of the parameters, slit speed, slit width and extrusion pressure.

In one embodiment the extruded layer of the composition has a uniform thickness. In an alternative embodiment the extruded layer has a variable thickness. Preferably, the total thickness of the layer(s) is from 3 mm to 5 cm, more preferably between 15-25mm. Preferably a single layer has a thickness of 7.5-12.5 mm.

In one embodiment of the invention, the composition is extruded on a surface. In a preferred embodiment the surface can be cooled. In an even more preferred embodiment the surface can cool the composition below freezing temperature.

The extrusion might occur at any temperature suitable for the biopolymer. In one embodiment of the invention the extrusion is performed at room temperature. In a preferred embodiment the extrusion is performed at 0 to + 40 °C. In one embodiment, the surface or mold has a temperature between -80 and +40 °C.

In a preferred embodiment the device the surface is a mold. In a more preferred embodiment the mold or surface can be cooled or refrigerated. In a more preferred embodiment the surface or mold is able to freeze a composition. In one preferred embodiment the surface or mold is capable to freeze a composition at high cooling rate. In an alternative preferred embodiment the surface or mold is capable to freeze at low cooling rate. In a most preferred embodiment the surface or mold is capable to provide variable time-depending cooling rates.

A variable cooling rate within the meaning of this invention refers to a cooling rate of 40 to 300 °C/h. In a preferred embodiment, the cooling rate is from 60 to 250 °C/h, more preferably from 100 to 200 °C/h.

The mold might have any shape, though a rectangular shape is preferred. In a preferred embodiment the length of the slit for extrusion is adapted to the mold.

In one embodiment of the invention the biocompatible matrix is based on a single layer. In an alternative embodiment the matrix is based on multiple layers. In a particular embodiment of the invention the method involves the repetition of the extrusion step to add one or more further layers.

Further layers might be added the same way as the first layer, preferably by extrusion through a slit. Said layers might consist of an identical composition as the first layer or consist of a different composition. In one embodiment of the invention all layers comprise the same biopolymer. In an alternative embodiment, at least one layer comprises a different biopolymer. Extrusion of different layers can be achieved by using an extrusion device with separate slits or separate extrusion devices.

Extrusion into a layer can be performed simultaneously to cooling or freezing of the nascent layer or the extruded layer can be cooled or frozen after completion of extrusion of said layer. Further layers might be added before or after complete freezing of respective previous layers.

The properties of the biocompatible matrix might be adjusted by the composition of additional layers. As such, further layers might be based on different biopolymers than those of the respective previous layer. In one embodiment of the invention all layers comprise the same biopolymer. In an alternative embodiment at least one layer comprises a different biopolymer.

Even if the biopolymers in the layers are identical, the compositions of the layers might be different in order to adapt matrix properties to the future use of the matrix. As such, the different layers might be based on the same basic biopolymer, but for example, one layer comprises a compound with antibiotic activity, and a second layer might only comprise a coloring agent.

Depending on the composition and intended use of the matrix, further layers might be added in different orientations than the first layer (see figure 2).

### Figure 2 shows:

1 Extrusion device
   1.1 Inlet port for composition
   1.2 Slit variable in size and shape
   1.3 Movement of extrusion device
2 Mold variable in size and shape
   2.1 Bottom surface of mold variable in topography, optionally cooled
3 Device allowing variable movement of mold, e.g. motor
   3.1 Motor with rotational movement
   3.2 Optional surface, e.g. table for mold. Surface is movable by 3 and optionally cooled
   3.3 Device of 3 used for rotational movement of mold for 90° relative to initial mold orientation
4 First layer prepared by extrusion of composition through slit
   4.1 First layer obtained by longitudinal direction of extrusion relative to initial mold orientation
5 Second layer deposited on top of the first layer by extrusion of composition through slit
   5.1 Second layer obtained by orthogonal/transverse direction of extrusion relative to initial mold orientation

In one embodiment of the invention, all layers are extruded in the same orientation relative to the first layer. In an alternative embodiment one or more layers are extruded orthogonal to the first layer.

The method allows the creation of improved one-dimensional tensile strength. By adapting the orientation of further layers, the tensile strength in multiple directions can be increased. Preferably the composition of the layers and the biopolymer in each layer are identical for multiple layers. In one embodiment the compositions comprising the at least one biopolymer are identical for all layers.

The inventors found that it is essential to freeze the extruded composition in the layer prior to an optional drying step. It is important that, e.g. if lyophilization is used for drying, the composition is frozen beforehand and not at the same time.

If the composition comprises multiple layers, it is possible to extrude and deposit all layers and freeze the whole base matrix or to extrude and freeze single layers. In one embodiment of the invention, each layer is frozen after extrusion before a new layer is extruded. In an alternative embodiment several layers are extruded and the composition is frozen after at least two layers have been extruded. After freezing of the first layers, subsequent layers might be added and frozen in any order. Hereby, the expression 'freezing' also comprises partial freezing, i.e. that liquid material is remained in a partially frozen layer, ensuring proper adhesion to other layers.

The inventors found that the freezing process influences the properties of the matrix. In one embodiment, the composition is shock-frozen after extrusion. In an alternative embodiment, the composition is slowly frozen after extrusion.

Cooling or freezing can be performed with low or high cooling rates. Modification of the cooling-or freezing-kinetics enables the adaption of crystal size. Higher cooling rates result in smaller crystals. Crystal size determines the later porosity of the biocompatible matrix.

The freezing process might be performed by any suitable process. In a preferred embodiment the composition is extruded onto a cooled surface or in a cooled mold. In an alternative embodiment the composition is extruded in a mold and then frozen in a freezer providing a matrix/air interface or in liquid nitrogen.

After freezing the extruded composition has to be dried. Any drying process is suitable; however, the inventors found that lyophilization enhances the properties of the matrix compared to other drying methods. In a preferred embodiment drying is performed with lyophilization.

In one embodiment of the invention the matrix is processed prior to drying. In one embodiment said processing involves mechanical shaping of frozen layer or whole base matrix. In a particular embodiment mechanical shaping is performed by means of plaining, slitting, cutting, cryo-cutting, die-cutting, CNC milling, grinding, generation of holes.

Lyophilization of the compositions provides the additional advantage, that the composition maintains the basic shape and alignment of the characteristic structure of the biopolymer as well as its authentic structural properties.

Targeted adaption of the lyophilisation process parameters is essential for determining later characteristics of the biocompatible matrix. For example, lyophilisation temperature has an impact on dehydrothermal crosslinking density resulting in different mechanical (e.g. tensile strength, elastic modulus) and biological properties (e.g. cell migration, biodegradability).

In an alternative embodiment the biocompatible matrix is dried by thermic drying.

After lyophilization, it is preferred that the dried composition is subjected to a crosslinking agent.

In case an additional chemical crosslinking step is performed after lyophilisation, the dehydrothermal crosslinking during lyophilisation is defined as dehydrothermal pre-crosslinking whereas subsequent additional chemical crosslinking is defined as chemical post-crosslinking. With reference to the desired performance parameters of the biocompatible matrix dehydrothermal pre-crosslinking and chemical post-crosslinking can be balanced to achieve a synergistic optimum.

In a preferred embodiment a pharmaceutically and/or cosmetically acceptable crosslinker is used.

Preferably the crosslinker is selected from the group comprising Di- or polyepoxides, preferably di or polyglycidyl structures, glycol diglycidylether, glycerol diglycidylether, butanediol diglycidylether, resorcinol diglycidylether, 1,6-hexanediol diglycidylether, ethylenglycol diglycidylether, di or, oligo or poly ethylenglycol diglycidylether, propylenglycol diglycidylether, di, oligo or polypropylenglycol diglycidylether, and polybutadiene diglycidylether, most preferably 1,4-butanediol diglycidylether (BDDGE), tri or polyfunctional epoxides, glycerol polyglycidylether, pentaerythritol polyglycidylether, di, oligo or polyglycerol polyglycidyl ether and trimethylolpropane polyglycidyl ether.

In a preferred embodiment the crosslinker is 1,4-Butanediol diglycidylether (BDDGE).

In a particular embodiment of the invention a second crosslinker is used or after chemical post-crosslinking the matrix is subjected to another crosslinking step.

The chemically post-crosslinked matrices are already suitable for many different applications. As such, in one embodiment of the invention, the post-crosslinked matrix is not modified after the crosslinking step. In an alternative embodiment the post-crosslinked matrix is physically modified, i.e. cut or sliced after the crosslinking step.

However, the inventors found that the properties of the matrix can be additionally improved, if the matrix is subjected to mechanical force before and/or during the chemical post-crosslinking.

Any kind of mechanical force is suitable and will modify the properties of the matrix. In one embodiment, mechanical force is applied by compressing the matrix. In a preferred embodiment mechanical force is applied by compressing the matrix between two plates. In a more preferred embodiment, mechanical force is applied by compressing the matrix between two plates, wherein the sites of the matrix are in contact with a plate comprising a specific 3D structure.

In an alternative preferred embodiment, the mechanical forces are applied by stretching the matrix in a frame.

The mechanical forces may be applied at room temperature. However, depending on the biopolymer different temperatures might be suitable. In a preferred embodiment the mechanical forces are applied at a temperature between - 20 and + 65 °C, preferably between 0 to 40 °C.

While mechanical forces are applied, the matrix might be further modified. One possible modification is a further crosslinking step. Additional modifications include mechanical treatments by means of slicing, cutting, die-cutting, embossing, slitting, generation of holes.

The present invention further relates to biocompatible matrices obtainable by the above described method.

Said biocompatible matrices may comprise one or more layers consisting of a composition comprising a biopolymer as described above.

The inventors found that biocompatible matrices obtained by a method according to the invention show improved properties, when compared to matrices of prior art. For example, a collagen based matrix, produced with methods according to the invention comprises improved tensile strength and reduced plastic deformation.

The inventors found in particular that matrices based on fiber- and/or fibril forming biopolymers show significantly improved properties, as the extrusion through the slit generates an alignment of the fiber-structure, thus improving properties like tear strength, elastic modulus, elongation and plastic deformation.

Other properties of the matrix might be enhanced or modified depending on the selected biopolymer and other compounds in the composition or compositions, if the matrix is based on more than one layer. Among the modifiable properties are biocompatibility, increased or reduced biodegradability and modified cell migration properties.

The invention further relates to the use of a matrix according to the invention as a medical device or cosmetic product.

Preferably matrices according to the invention are used in fields of clinical applications selected from the group comprising abdomen, breast, tendon, rotator cuff, ligament, ocular, pericard, dura mater, artery, dental, facial, trauma surgery, minimally invasive surgery.

The matrices according to the invention are suitable for clinical treatment goals selected from the group comprising mechanical support, mechanical reinforcement, augmentation, repair, reconstruction, regeneration, defect closure, promotion of healing.

A most preferred use for a matrix according to the present invention is the use as a medical device, preferably as an implant. In a more preferred embodiment the matrix according to the invention is used as a biodegradable implant.

### Examples

### Targeted manufacturing of biocompatible matrices with adapted biomechanical properties by directed extrusion, freezing and lyophilisation

Collagen is extracted and purified from bovine dermis by means of acidic, alkaline and oxidative treatment. The purified collagen is minced and milled to a homogeneous collagen dispersion of medium viscosity. The collagen dispersion is used as composition for extrusion at constant flow rate and slit movement velocity at room temperature producing collagen layers according to the invention. Molds defining later matrix shape and orientation are placed longitudinally or orthogonally to slit movement direction. Layers are frozen at -45 °C and lyophilized. Specimen for biomechanical testing are cut out of the lyophilized layers.

Figure 3 shows biomechanical data of matrices obtained by longitudinal (dark grey) or orthogonal (light grey) extrusion with respect to the later matrix shape and orientation. Matrices show significant differences regarding the biomechanical performance parameters tensile strength, elongation and elastic modulus. Matrices obtained by longitudinal extrusion show a higher tensile strength compared to matrices produced by orthogonal extrusion. By longitudinal extrusion produced matrices are also stiffer and therefore possess a higher elastic modulus. The elongation at maximum force (Fₘₐₓ) is lower for these matrices.

The data clearly show that the properties of matrices produced according to the invention are adaptable for optimized performance in the desired field of application.

### Targeted manufacturing of biocompatible matrices with adapted biomechanical properties by directed extrusion, freezing and lyophilisation and chemical post-crosslinking with mechanical treatment

Collagen is extracted and purified from bovine dermis by means of acidic, alkaline and oxidative treatment. The purified collagen is minced and milled to a homogeneous collagen dispersion of medium viscosity. The collagen dispersion is used as composition for extrusion at constant flow rate and slit movement velocity at room temperature producing collagen layers according to the invention. Molds defining later matrix shape and orientation are placed longitudinally or orthogonally to slit movement direction. Layers are frozen at -45 °C and lyophilized. Lyophilized layers were mounted between steel frames and exposed to aqueous crosslinker solution containing BDDGE. After subsequent washing with water and air drying at room temperature specimen for mechanical testing were cut out of the resulting layers.

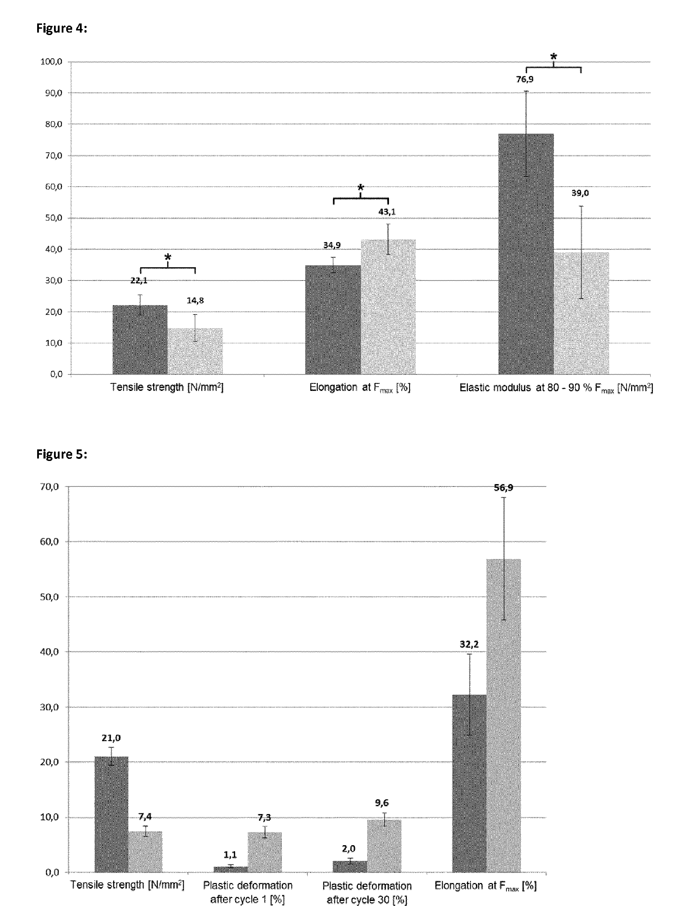

Figure 4 shows biomechanical data of such chemically post-crosslinked matrices obtained by longitudinal (dark grey) or orthogonal (light grey) extrusion with respect to the later matrix shape and orientation. Similar as the matrices of figure 3, this matrices show significant differences regarding their biomechanical performance parameters. Again, matrices obtained by longitudinal extrusion show a higher tensile strength, a higher elastic modulus and a lower elongation at maximum force (Fₘₐₓ) compared to matrices produced by orthogonal extrusion. Interestingly the values for all parameters could be increased by additional post-crosslinking with mechanical treatment compared to matrices produced according to figure 3. Therefore the innovative production process is not only capable of generating biocompatible matrices with high tensile strength, it is also able to fine tune their characteristics on different production process levels (i.e. extrusion orientation and freezing, lyophilization (including adaption of dehydrothermal pre-crosslinking), chemical post-crosslinking with optional mechanical treatment) for optimal targeted product performance.

The properties of a chemically post-crosslinked matrix according to the present invention compared with a matrix of prior art are shown in figure 5. It is clearly visible, that the innovative production process including directed extrusion, freezing, lyophilization with dehydrothermal pre-crosslinking and subsequent chemical post-crosslinking with mechanical treatment improves the properties of the resulting matrix drastically, compared to the prior art matrices. The tensile strength of matrices according to the present invention can be increased significantly compared to the matrices of prior art. Also the plastic deformation (i.e. irreversible elongation, which is not desired for most matrices for use as medical device, especially as implant) after 1 or 30 cycles of force and force removal is much lower for matrices obtained according to the present invention. The data clearly show that the method according to the invention is generating new matrices with greatly improved properties, which are suitable for a wide array of applications.

### Figure legends

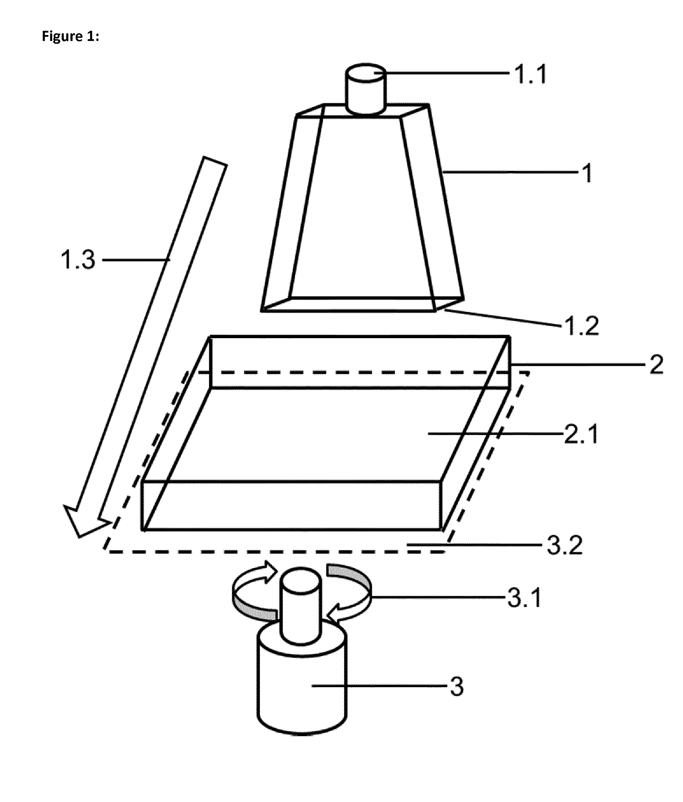

**Figure 1****:** Schematic representation of an extruding method according to the invention. The extrusion device (1) is provided with a composition through the inlet port (1.1). The composition is extruded through a slit (1.2) into a mold (2), wherein the slit moves in a straight line over the mold. The bottom surface of the mold is variable in topography and optionally cooled. A device allowing variable movement of mold (3), e.g. a motor with rotational movement (3.1) enables extrusion of the composition in variable directions with regard to the later matrix shape and orientation defined by the mold. The mold can be placed onto an optional surface, e.g. a table for mold (3.2). The surface is movable by (3) and optionally cooled.
1 Extrusion device
   1.1 Inlet port for composition
   1.2 Slit variable in size and shape
   1.3 Movement of extrusion device
2 Mold variable in size and shape
   2.1 Bottom surface of mold variable in topography, optionally cooled
3 Device allowing variable movement of mold, e.g. motor
   3.1 Motor with rotational movement
   3.2 Optional surface, e.g. table for mold. Surface is movable by 3 and optionally cooled

**Figure 2****:** Schematic representation of an extruding method according to the invention. The extrusion device (1) is provided with a composition through the inlet port (1.1). The composition is extruded through a slit (1.2) into a mold (2), wherein the slit moves in a straight line over the mold. The bottom surface of the mold is variable in topography and optionally cooled. A device allowing variable movement of mold (3), e.g. a motor with rotational movement (3.1) enables extrusion of the composition in variable directions regarding the later matrix shape and orientation defined by the mold. The mold can be placed onto an optional surface, e.g. a table for mold (3.2). The surface is movable by (3) and optionally cooled. According to the invention extruding of separate layers is possible by extrusion in different orientations.
A) A first layer (4.1) is obtained by longitudinal direction of extrusion relative to initial mold orientation.
(B) After 90° mold rotation, a second layer (5.1) is extruded onto the first layer in orthogonal orientation.
   1 Extrusion device
      1.1 Inlet port for composition
      1.2 Slit variable in size and shape
      1.3 Movement of extrusion device
   2 Mold variable in size and shape
      2.1 Bottom surface of mold variable in topography, optionally cooled
   3 Device allowing variable movement of mold, e.g. motor
      3.1 Motor with rotational movement
      3.2 Optional surface, e.g. table for mold. Surface is movable by 3 and optionally cooled
      3.3 Device of 3 used for rotational movement of mold for 90° relative to inital mold orientation
   4 First layer prepared by extrusion of composition through slit
      4.1 First layer obtained by longitudinal direction of extrusion relative to initial mold orientation
   5 Second layer deposited on top of the first layer by extrusion of composition through slit
      5.1 Second layer obtained by orthogonal/transverse direction of extrusion relative to initial mold orientation

**Figure 3****:** Comparison of physical properties of a matrix obtained by extruding the composition longitudinally (dark grey) or orthogonally (light grey) according to the invention and subsequent freezing and lyophilisation.

**Figure 4****:** Comparison of physical properties of a matrix obtained by extruding the composition longitudinally (dark grey) or orthogonally (light grey) and subsequent freezing, lyophilisation and chemical post-crosslinking with mechanical treatment according to the invention.

**Figure 5****:** Comparison of physical properties of a matrix obtained by extruding the composition longitudinally (dark grey) and subsequent freezing, lyophilisation and chemical post-crosslinking with mechanical treatment according to the invention in comparison to a benchmark produced according to prior art (light grey).

## Claims

1. A method for the production of a biocompatible matrix with adaptable properties the method comprising:
a) providing a composition comprising at least one biopolymer;
b) extruding the composition into a layer through a slit onto a surface, wherein the slit moves over the surface;
c) freezing the composition after extrusion;
d) optionally repeating the process to add one or more further layers;
e) lyophilizing the frozen composition;
f) optionally subjecting the composition to mechanical force.
g) crosslinking the at least one biopolymer;
wherein preferably the surface can be cooled.

2. A method according to claim 1, wherein the composition comprising a biopolymer further comprises proteins, glycoproteins, proteoglycans polysaccharides and/or glucosaminoglycans.

3. A method according to any of claims 1 and 2, wherein the composition comprising at least one biopolymer is of biological or biotechnological origin, a biologically derived tissue or tissue compartment.

4. A method according to any of claims 1 to 3, wherein the composition comprising at least one biopolymer is a biologically derived tissue selected from the group of origin comprising bovine, equine, porcine, rodent, piscine tissue, preferably skin, tendon or cartilage tissue, most preferably bovine split skin.

5. A method according to any of claims 1 to 4, wherein the at least one biopolymer is collagen.

6. A method according to any of claims 1 to 5, wherein the thickness of the layer is determined by the width of the slit and/or the movement speed of the slit relative to the surface.

7. A method according to any of claims 1 to 6, wherein the steps a) to c) are repeated at least once, wherein in the repeated process a second composition comprising at least one biopolymer is used.

8. A method according to any of claims 1 to 7, wherein the steps a) to c) are repeated at least once, wherein further layers can be extruded in a different orientation compared to the respective previous layers.

9. A method according to any of claims 1 to 8, wherein the crosslinking is performed with a crosslinker selected from the group comprising Di- or polyepoxides, preferably di or polyglycidyl structures, glycol diglycidylether, glycerol diglycidylether, butanediol diglycidylether, resorcinol diglycidylether, 1,6-hexanediol diglycidylether, ethylenglycol diglycidylether, di or, oligo or poly ethylenglycol diglycidylether, propylenglycol diglycidylether, di, oligo or polypropylenglycol diglycidylether, and polybutadiene diglycidylether, most preferably 1,4-butanediol diglycidylether (BDDGE), tri or polyfunctional epoxides, glycerol polyglycidylether, pentaerythritol polyglycidylether, di, oligo or polyglycerol polyglycidyl ether and trimethylolpropane polyglycidyl ether.

10. A method according to any of claims 1 to 9, wherein the crosslinking is performed at a pH between 2 and 9.

11. A method according to any of claims 1 to 10, wherein in step g) the mechanical forces are applied by stretching the composition in a frame.

12. A method according to any of claims 1 to 11, wherein step g) additionally comprises an additional crosslinking step.

13. A method according to any of claims 1 to 12, wherein step g) is performed at a temperature between - 20 °C and +60 °C, preferably between 0 to 40 °C.

14. A biocompatible matrix obtainable by a process according to any of claims 1 to 13.

15. Use of a biocompatible matrix according to claim 14 as a cosmetic product or medical device.
